# EUROPEAN PATENT SPECIFICATION

(11) **EP 4 648 707 B1**
(45) Date of publication and mention of the grant of the patent: **25.03.2026**
(21) Application number: 24817981.4
(22) Date of filing: 04.12.2024
(51) Int. Cl.: A61B 90/00, A61B 17/00, A61B 34/20, A61B 90/96, A61B 90/98, A61B 90/50

(54) **UNIVERSAL TRACKING MARKER INTERFACE COMPONENT**
UNIVERSELLE VERFOLGUNGSMARKERSCHNITTSTELLENKOMPONENTE
COMPOSANT D'INTERFACE DE MARQUEUR DE SUIVI UNIVERSEL

(30) Priority: 04.12.2023 WO PCT/EP2023/084145
(43) Date of publication of application: 19.11.2025
(73) Proprietor: Brainlab SE, 81829 München (DE)
(72) Inventor: LENNERTZ, Philippe, Munich (DE); VUILLEMIN, Jerome, Munich (DE); HOFBERGER, Stefan, Munich (DE)
(74) Representative: SSM Sandmair
(86) International application number: PCT/EP2024/084609
(87) International publication number: WO 2025/119949

(56) References cited:
- US-A1- 2008 200 794
- US-A1- 2012 168 587
- US-A1- 2015 297 315
- US-A1- 2021 244 478
- US-A1- 2023 047 595
- US-A1- 2023 235 765

## Description

### FIELD OF THE INVENTION

The present invention relates to a tracking marker interface component which is used for mounting tracking markers to medical instruments and devices in a statically determined manner for tracking and navigation purposes. In particular, the tracking marker interface component may be used for mounting various types of tracking markers to instruments and devices so as to cover a variety of medical and surgical use cases and to enable the use of various tracking technologies.

### TECHNICAL BACKGROUND

In computer-assisted medical procedures and surgical interventions, practitioners and surgeons use instruments and devices which are specifically adapted to be tracked by a medical navigation system. The relative position of these instruments can therefore be determined with respect to a patient's anatomy, wherein the instrument's spatial position (spatial location and/or spatial orientation) with respect to an image representation of the patient's anatomy is shown to the practitioner or surgeon via a display. It is common for trackable instruments and devices to include a specific type of tracking marker which is spatially recognized by a corresponding medical tracking system. A change in the tracking technology and even a change in the instrument's use case requiring a different tracking technology therefore requires entirely new instruments and devices to be developed and approved. US 2023/0235765 discloses interface components according to the preamble of independent claim 1. US 2008/0200794, US 2021/0244478 and US 2015/0297315 disclose various interface components for mounting medical tracking markers to structures.

The present invention has the object of providing a universal tracking marker interface which allows instruments and devices to be spatially tracked via a variety of different tracking technologies.

The present invention can be used for any medical and surgical procedures involving the use of medical tracking and navigation systems such as Curve^{®} and Kick^{®}, both products of Brainlab AG.

Aspects of the present invention, examples and exemplary steps and their embodiments are disclosed in the following. Different exemplary features of the invention can be combined in accordance with the invention wherever technically expedient and feasible.

### EXEMPLARY SHORT DESCRIPTION OF THE INVENTION

In the following, a short description of the specific features of the present invention is given which shall not be understood to limit the invention only to the features or a combination of the features described in this section.

The present invention provides a tracking marker interface component which may connect to a correspondingly shaped counterpart interface component in a releasable and/or statically determined manner. In particular, the interface component may connect to such correspondingly shaped counterpart interface component in a reproducible manner, which means that corresponding first and second interface components will, as soon as they connect to each other, always take the identical relative position with respect to each other.

### GENERAL DESCRIPTION OF THE INVENTION

In this section, a description of the general features of the present invention is given for example by referring to possible embodiments of the invention.

In general, the invention reaches the aforementioned object by providing, in a first aspect, a tracking marker interface component comprising at least one first positioning section, at least one second positioning section and at least one fixation section. In this context, statically determined means that the respective interface components connect to each other in a manner without "forcing" the interface components into place, which may result in elastic deformations of one or both interface components. In particular, the interface component is adapted to connect to a correspondingly shaped counterpart interface component with (exactly) all six degrees of freedom (three rotational degrees of freedom and three translational degrees of freedom within the three dimensional space) being determined. For example, assuming the interface components experience different thermal expansions, which may result from one of the interface components expanding faster and/or by a higher amount when being exposed to an increase of temperature, none of the interface components will undergo a constraining stress because of how the interface components connect to each other. This will be described in more detail below.

According to the invention, the interface component is adapted to be brought into contact with and separated from a counterpart interface component substantially along a first spatial axis. The fixation section is further adapted to prevent the interface component from being separated from a counterpart interface component. In other words, the interface component may be brought into contact with a counterpart interface component by a translational motion substantially along a first spatial axis, wherein no additional rotational or translational motion is necessary for bringing the interface components into contact with each other. The same may apply for disconnecting and separating the interface components from each other.

While the first positioning section features a profile that extends along a third spatial axis perpendicular to the first spatial axis, the second positioning section features a profile that extends along a second spatial axis perpendicular to the first spatial axis and the third spatial axis.

Further, the corresponding first positioning section may be adapted to prevent a translational relative motion of the interface component when contacting a counterpart interface component along a second spatial axis perpendicular to the first spatial axis, and to allow for a translational relative motion along a third spatial axis perpendicular to the first spatial axis and to the second spatial axis, wherein the corresponding second positioning section may be adapted to prevent a translational relative motion of the interface component when contacting a counterpart interface component along the third spatial axis, and to allow for a translational relative motion along the second spatial axis. It becomes apparent that it is the first and the second positioning sections, respectively, which ensure that the interface components connect to each other in the above described statically determined manner. In this regard, each one of the first positioning section and the second positioning section prevent, when the interface components connect to each other, one translational relative motion between the interface components, wherein the directions of these translational motions extend perpendicularly to each other as well as to the direction in which a translational relative motion between the interface components is prevented by the fixation sections. As each one of the first positioning section, the second positioning section and the fixation section prevents a translational motion along one of a total of three spatial directions, any translational motion of the interface component is prevented once the interface component connects to a correspondingly shaped counterpart interface component. In this regard, it should be noted that the fixation section, the first positioning section and the second positioning section prevent for a translational motion in one spatial direction only so as to prevent constraint stress and to allow for a statically determined connection between the interface components. It will be understood that any rotational motion between the interface components may also be prevented by the fixation section, the first positioning section and the second positioning section being spaced apart from each other. Still, any one of the fixation section, the first positioning section and the second positioning section may as such be adapted or formed to prevent a rotational motion of interface component with respect to a correspondingly shaped counterpart interface component.

For example, at least one of the first and second positioning sections is shaped as a protrusion protruding from the interface component substantially along the first spatial axis. Further, at least one of the first and second positioning sections may also be shaped as a recess substantially extending along the first spatial axis. In other words, any of the first and second positioning sections may be shaped as a male or as a female connector. In this regard, the positioning sections may comprise shaped surfaces which may generally allow for an areal contact to correspondingly shaped counterpart positioning sections. It is however also conceivable that the positioning sections are shaped to substantially allow for one or more "linear contacts" to correspondingly shaped counterpart positioning sections. This means that the positioning sections may be generally shaped for physical contact along one or more lines across the positioning section's surfaces. For example, these one or more contact lines may extend along the spatial direction in which the respective positioning sections allow for a translational motion of the interface component.

In a more specific example, the protrusion mentioned above comprises at least one convex, particularly cylindrically shaped surface section and/or the recess comprises at least one concave or prismatically shaped surface section. In case the positioning sections are represented by a convex protrusion or a concave recess having the same curvature as a correspondingly shaped counterpart positioning section, one can easily imagine that the corresponding positioning sections may contact each other substantially across an areal surface area. A difference in the curvature of the recess and the curvature of the protrusion may result in the corresponding positioning sections contacting each other along one or more lines. The same would be the case if one of the recess and the protrusion includes a curved surface, for example a cylindrical surface, whilst the other of the recess and the protrusion includes a prismatically shaped surface, i.e. includes a plurality of flat surface sections adjoining each other at an angle.

In general, each one of the first positioning sections and the second positioning sections may include rail-shaped protrusions and/or correspondingly shaped grooves extending along the spatial direction in which the respective positioning sections allow for a translational motion of the interface component.

For example, the first positioning section of the interface component includes, particularly consists of two protruding surface sections which extend coaxially and substantially along the second spatial axis, particularly which extend from the interface component in substantially opposite directions. In a further example, any of the rail-shaped protrusion and the groove-shaped recess may be interrupted by any conceivable intermediate structure, resulting in respectively shaped end-sections which however have the same functionality as an uninterrupted rail or groove.

In another example, the second positioning section of one of the first interface component and second interface component includes, particularly consists of one protruding surface section, which extends substantially perpendicularly to the surface section(s) of the first positioning section, specifically along a plane perpendicular to the first spatial axis and defined by the surface section(s) of the first positioning section. From this specific example, it becomes apparent that the first and the second positioning sections are formed by rail-groove-pairings which extend perpendicularly to each other. Thus, the translational motion between the interface components which would be allowed for by one of the positioning sections is prevented by the respective other positioning section, and vice versa, thereby allowing for a statically determined connection between the interface components. In this regard, it further needs to be noted that the first and the second positioning sections do not necessarily extend along or within the same plane, but may also extend along or within parallel planes, both of which may be perpendicular to the first spatial axis. Moreover, the first and the second positioning sections may even extend, for example to some degree and/or due to manufacturing tolerances, in non-parallel planes which are not perfectly perpendicular to the first spatial axis.

Further, the fixation section of the interface component is configured to establish a friction fit and/or a form fit. In this regard, any suitable friction-fit or form-fit connection is conceivable for releasably coupling the interface component to a counterpart interface component. For example, the interface component may include a threaded hole or bore for receiving a threaded bolt, or vice versa, both of which may extend along the first spatial axis. Additionally or alternatively, the interface component may include a connector portion having an undercut extending perpendicularly to the first spatial direction, particularly an elastically deformable snap-in-connector. As will be described further below, the interface component may comprise more than one fixation-section, for example a thread connection and a snap-in-connection. This allows for connecting interface components of a different type, for example connecting an interface component made from metal to an interface component made from plastic. While it may be beneficial for an interface-component-pairing with both interface components being made from metal to have corresponding threaded fixation sections, an interface-components-pairing with both of the interface components being made from plastic may benefit from a snap-in-type of fixation sections because of manufacturing reasons. If both or even more types of fixation sections is provided by the respective interface components, any conceivable pairing of interface components is conceivable, even if the interface components are of a different type.

In another, even more specific example, the fixation section of one of the first interface component and second interface component includes
- an internal thread adapted to engage with a screw assigned to the other of the first interface component and second interface component; and/or
- an elastically deformable snap-in connector adapted to engage with, particularly reach behind an edge of the other of the first interface component and second interface component; and/or
- a permanent magnet or electromagnet adapted to attract a corresponding permanent magnet, electromagnet or ferromagnetic element of the other of the first interface component and second interface component.

Further, the interface component may connect to, particularly may be integrally formed with a tracking marker adapted to be recognized and spatially tracked by a medical tracking system. The interface component may also connect to, particularly may be integrally formed with a medical instrument or device.

In a further example, at least one of the first positioning section and the second positioning section comprises an at least sectionwise constant profile along the third spatial axis and the second spatial axis, respectively. In other words, one or more portions of a positioning section may feature a substantially identical profile or shape or surface along the axis in which they extend.

In a further example, at least one of the first positioning section and the second positioning section comprises at least two sections having an identical profile along the third spatial axis and the second spatial axis, respectively. In other words, any of the positioning sections may feature at least two portions that have a substantially identical profile or shape or surface along the axis in which they extend, while other portions may have a profile or shape or surface that differs therefrom.

Moreover, the fixation section may be disposed/positioned between the first positioning section and the second positioning section. This may help in distributing applied retaining forces more evenly to the positioning sections.

In a still other example, the interface component may be made from plastic and/or may be configured as disposable article to be distributed pre-sterilized. In particular, the interface component may be injection molded and particularly configured
- as undercut-free structure;
- to be molded in a slider-free injection-mold;
- to be molded in an injection-mold having one single unmolding direction; and/or
- to be demolded without elastic deformation.

In this regard and in order to avoid repeated use of the interface component which impairs sterility in a surgical environment, the interface component is configured as disposable article and comprises, particularly at the fixation section, a predetermined breaking section that disables functionality of the interface component, particularly of the fixation section, when being fractionized. In other words, the measures described in the following are to prevent a repeated use of the interface component. This may be achieved by causing certain parts of the interface component to lose their functionality once the interface component is removed from its counterpart interface component and/or when the engaging state of the fixation section is rescinded by any means.

Further, the predetermined breaking section may be adapted to be fractionized by
- a force and/or a moment exceeding a predefined amount and acting on the fixation section along the first spatial axis;
- a force and/or a moment exceeding a predefined amount and acting on the fixation section in a direction opposite to an engagement direction in which the fixation section is configured to move for establishing the positive fit and/or the friction fit.

The breaking section may connect between the interface component, particularly the fixation section, and an element configured to be detached from the interface component, particularly the fixation section, wherein the detachable element is in particular adapted to be torn off or twisted off the interface component, particularly the fixation section.

In particular, the detachable element may be configured to prevent the fixation section to move in a direction opposite to an engagement direction in which the fixation section is configured to move for establishing the positive fit and/or the friction fit.

Further, the interface component may be made from metal and/or may be configured as reusable and sterilizable article. In particular, the interface component may be machined, particularly involving a milling-process.

A further aspect of the present invention relates to the use of a tracking marker interface according to any embodiment described herein for connecting, particularly in a statically determined manner, a medical tracking marker to a medical instrument or medical device, particularly by bringing the tracking marker interface component into contact with a correspondingly shaped counterpart interface component.

### DEFINITIONS

In this section, definitions for specific terminology used in this disclosure are offered which also form part of the present disclosure.

### Marker

It is the function of a marker (i.e. tracking marker) to be detected by a marker detection device (i.e. tracking system, for example, a camera or an ultrasound receiver or analytical devices such as CT or MRI devices) in such a way that its spatial position (i.e. its spatial location and/or alignment) can be ascertained. The detection device is for example part of a navigation system. The markers can be active markers. An active marker can for example emit electromagnetic radiation and/or waves which can be in the infrared, visible and/or ultraviolet spectral range. A marker can also however be passive, i.e. can for example reflect electromagnetic radiation in the infrared, visible and/or ultraviolet spectral range or can block X-ray radiation. To this end, the marker can be provided with a surface which has corresponding reflective properties or can be made of metal in order to block the X-ray radiation. It is also possible for a marker to reflect and/or emit electromagnetic radiation and/or waves in the radio frequency range or at ultrasound wavelengths. A marker preferably has a spherical and/or spheroid shape and can therefore be referred to as a marker sphere; markers can however also exhibit a cornered, for example cubic, shape.

### Marker array

A marker array can for example be a reference star or a pointer or a single marker or a plurality of (individual) markers which are then preferably in a predetermined spatial relationship. A marker array comprises one, two, three or more markers, wherein two or more such markers are in a predetermined spatial relationship. This predetermined spatial relationship is for example known to a navigation system and is for example stored in a computer of the navigation system.

In another embodiment, a marker array comprises an optical pattern, for example on a two-dimensional surface. The optical pattern might comprise a plurality of geometric shapes like circles, rectangles and/or triangles. The optical pattern can be identified in an image captured by a camera, and the position of the marker array relative to the camera can be determined from the size of the pattern in the image, the orientation of the pattern in the image and the distortion of the pattern in the image. This allows determining the relative position in up to three rotational dimensions and up to three translational dimensions from a single two-dimensional image.

The position of a marker array can be ascertained, for example by a medical navigation system. If the marker array is attached to an object, such as a bone or a medical instrument, the position of the object can be determined from the position of the marker array and the relative position between the marker array and the object. Determining this relative position is also referred to as registering the marker array and the object. The marker array or the object can be tracked, which means that the position of the marker array or the object is ascertained twice or more over time.

### Marker Interface

A marker interface is understood to mean an interface for an individual marker which serves to attach the marker to an instrument, a part of the body and/or a holding element of a reference star, wherein it can be attached such that it is stationary and advantageously such that it can be detached. A fixation section (such as for instance a latching mechanism) of the marker interface can be provided at the marker interface and assists in placing the marker array on an instrument and the like in a force fit and/or positive fit.

### Pointer

A pointer is a rod which may comprise one or more - advantageously, a single - marker fastened to it and which can be used to measure off individual co-ordinates, for example spatial co-ordinates (i.e. three-dimensional co-ordinates), on a part of the body, wherein a user guides the pointer (for example, a part of the pointer which has a defined and advantageously fixed position with respect to the at least one marker attached to the pointer) to the position corresponding to the co-ordinates, such that the position of the pointer can be determined by using a surgical navigation system to detect the marker on the pointer. The relative location between the markers of the pointer and the part of the pointer used to measure off co-ordinates (for example, the tip of the pointer) is for example known. The surgical navigation system then enables the location (of the three-dimensional co-ordinates) to be assigned to a predetermined body structure, wherein the assignment can be made automatically or by user intervention.

### Reference star

A "reference star" refers to a device with a number of markers, advantageously three markers, attached to it, wherein the markers are (for example detachably) attached to the reference star such that they are stationary, thus providing a known (and advantageously fixed) position of the markers relative to each other. The position of the markers relative to each other can be individually different for each reference star used within the framework of a surgical navigation method, in order to enable a surgical navigation system to identify the corresponding reference star on the basis of the position of its markers relative to each other. It is therefore also then possible for the objects (for example, instruments and/or parts of a body) to which the reference star is attached to be identified and/or differentiated accordingly. In a surgical navigation method, the reference star serves to attach a plurality of markers to an object (for example, a bone or a medical instrument) in order to be able to detect the position of the object (i.e. its spatial location and/or alignment). Such a reference star for example features a way of being attached to the object (for example, a clamp and/or a thread) and/or a interface/holding element which ensures a distance between the markers and the object (for example in order to assist the visibility of the markers to a marker detection device) and/or a marker interface which are mechanically connected to the interface/holding element and which the markers can be attached to.

### Navigation system

The present invention is also directed to a navigation system for computer-assisted surgery. This navigation system preferably comprises a computer for processing the data provided in accordance with the computer implemented method. The navigation system preferably comprises a tracking system, i.e. a detection device for detecting the position of detection points which represent the main points and auxiliary points, in order to generate detection signals and to supply the generated detection signals to the computer, such that the computer can determine the absolute main point data and absolute auxiliary point data on the basis of the detection signals received. A detection point is for example a point on the surface of the anatomical structure which is detected, for example by a pointer. In this way, the absolute point data can be provided to the computer. The navigation system also preferably comprises a user interface for receiving the calculation results from the computer (for example, the position of the main plane, the position of the auxiliary plane and/or the position of the standard plane). The user interface provides the received data to the user as information. Examples of a user interface include a display device such as a monitor, or a loudspeaker. The user interface can use any kind of indication signal (for example a visual signal, an audio signal and/or a vibration signal). One example of a display device is an augmented reality device (also referred to as augmented reality glasses) which can be used as so-called "goggles" for navigating. A specific example of such augmented reality glasses is Google Glass (a trademark of Google, Inc.). An augmented reality device can be used both to input information into the computer of the navigation system by user interaction and to display information outputted by the computer.

### Surgical navigation system

A navigation system, such as a surgical navigation system, is understood to mean a system which can comprise: at least one marker array; a transmitter which emits electromagnetic waves and/or radiation and/or ultrasound waves; a receiver which receives electromagnetic waves and/or radiation and/or ultrasound waves; and an electronic data processing device which is connected to the receiver and/or the transmitter, wherein the data processing device (for example, a computer) for example comprises a processor (CPU) and a working memory and advantageously an indicating device for issuing an indication signal (for example, a visual indicating device such as a monitor and/or an audio indicating device such as a loudspeaker and/or a tactile indicating device such as a vibrator) and a permanent data memory, wherein the data processing device processes navigation data forwarded to it by the receiver and can advantageously output guidance information to a user via the indicating device. The navigation data can be stored in the permanent data memory and for example compared with data stored in said memory beforehand.

### Fixed (relative) position

A fixed position, which is also referred to as fixed relative position, in this document means that two objects which are in a fixed position have a relative position which does not change unless this change is explicitly and intentionally initiated. A fixed position is in particular given if a force or torque above a predetermined threshold has to be applied in order to change the position. This threshold might be 10 N or 10 Nm. In particular, the position of a sensor device remains fixed relative to a target while the target is registered or two targets are moved relative to each other. A fixed position can for example be achieved by rigidly attaching one object to another. The spatial location, which is a part of the position, can in particular be described just by a distance (between two objects) or just by the direction of a vector (which links two objects). The alignment, which is another part of the position, can in particular be described by just the relative angle of orientation (between the two objects).

### BRIEF DESCRIPTION OF THE DRAWINGS

In the following, the invention is described with reference to the appended figures which give background explanations and represent specific embodiments of the invention. It should be noted here that in the following, for illustrative and explanatory reasons, reference is made to two interrelating interface components forming a tracking marker interface. The present invention however also relates to one of these interface components which may either connect to a tracking marker or to an instrument or device. The scope of the invention is however not limited to the specific features disclosed in the context of the figures, wherein
- Fig. 1: shows a surgical instrument along with different types of tracking markers, both featuring correspondingly formed interface components,
- Fig. 2: shows a first embodiment of correspondingly formed interface components,
- Fig. 3: shows a second embodiment of correspondingly formed interface components,
- Fig. 4: shows an injection molded interface component,
- Fig. 5: shows an interface component corresponding to the interface component shown in Fig. 4,
- Fig. 6: shows a 3D view of an interface component comprising an electrical connector for energy or data transmission,
- Fig. 7: shows the instrument of Fig. 1 along with a tracking marker connected to the instrument via correspondingly shaped tracking marker interface components,
- Fig. 8: shows further instruments and devices featuring an interface component, and
- Fig. 9A/9B: show further tracking markers featuring an interface component.

### DESCRIPTION OF EMBODIMENTS

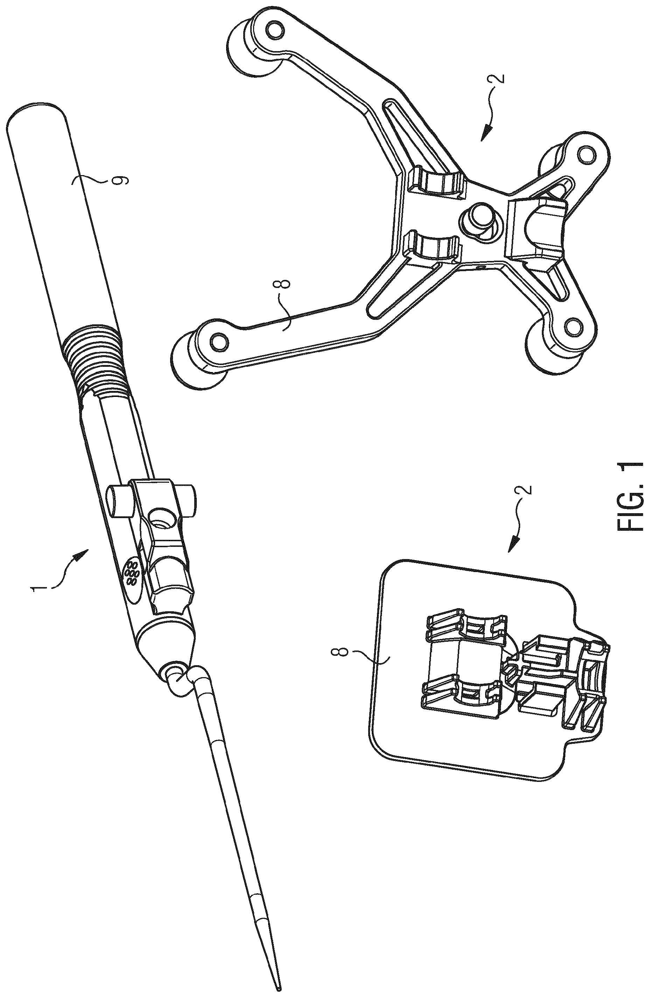

Figure 1 shows a pointer instrument 9 which is commonly used in surgical procedures for palpating objects and anatomical structures so as to determine their spatial position. While the pointer 9 includes a first interface component 1 being integrally formed with the pointer body, different types of tracking markers 8 provide a correspondingly shaped second interface component 2 adapted to releasably connect to the first interface component 1.

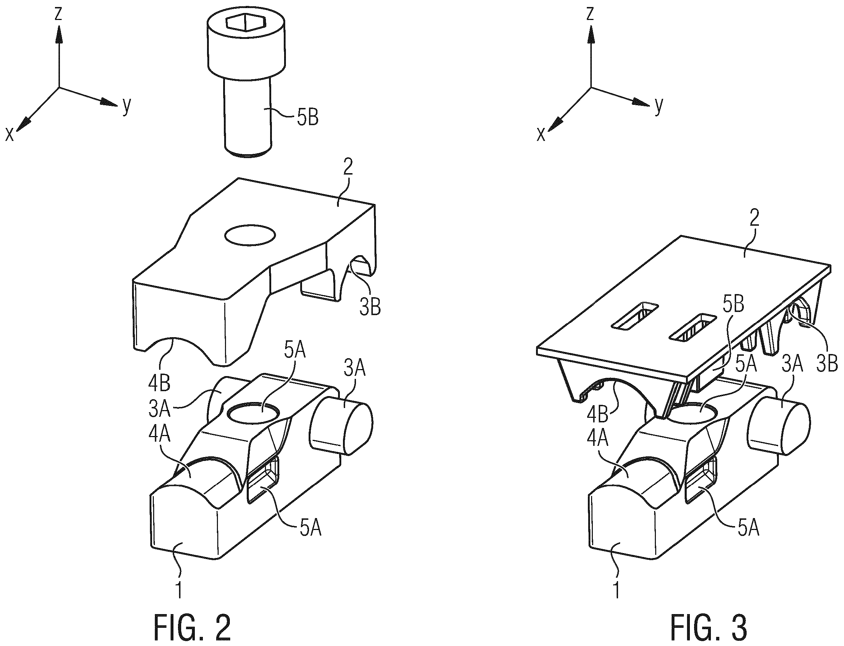

Figure 2 provides a more detailed view on a first interface component 1 and a second interface component 2 which are adapted to releasably and rigidly interlock in a statically determined manner. In the shown example, both of the first interface component 1 and second interface component 2 may be made from a metallic material and may therefore represent milled components. The first interface component 1 comprises two first positioning sections 3A which protrude in opposing directions from a central structure of the interface component 1, both of which have a cylindrical contact surface. Further, a second positioning section 4A also having a cylindrical contact surface is provided at a more distal position of the interface component 1. The correspondingly shaped second interface component 2 includes two first positioning sections 3B and one second positioning section 4B, all of which are formed as cylindrically shaped recesses for receiving the positioning sections 3A and 3B of the first interface component 1. Once the first interface component 1 and the second interface component 2 have been brought into engagement with each other, with the corresponding positioning sections 3A, 3B, 4A and 4B contacting each other, the second interface component 2 can be fastened to the first interface component 1 via a threaded bolt 5B screwed into the threaded bore 5A of the first interface component 1.

From Figure 2 it becomes apparent that, within the plane spanned by the directions X and Y, the pairing of the first positioning sections 3A and 3B as well as the pairing of the second positioning sections 4A and 4B allow for one translational motion between the interface components 1 and 2, while this motion is prevented by the respective other pairing. Further, a relative motion along the Z-direction is prevented by the fixation sections, i.e. the threaded bolt 5B engaging with the threaded hole 5A. As the positioning sections 3A, 3B, 4A and 4B are spaced apart from each other, any rotational relative motion between the interface components 1 and 2 is also prevented. As a result, all six degrees of freedom between the first interface component 1 and the second interface component 2 are set in a statically determined manner. For disconnecting the tracking marker 8 from the instrument 9, bolt 5B is unscrewed from hole 5A such that the second interface component 2 can be released from the first interface component 1.

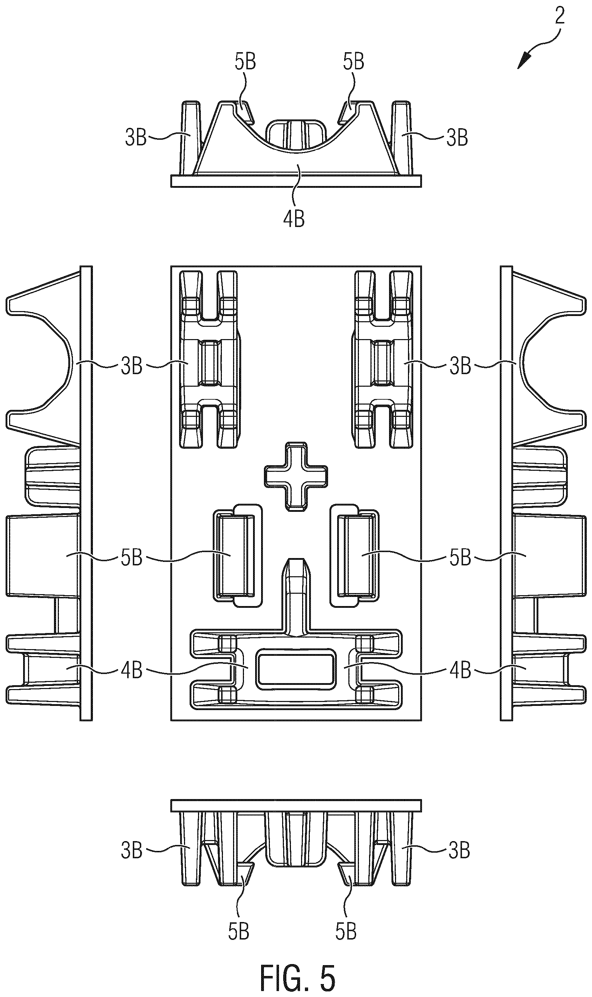

The embodiment shown in Figure 3 has the same functionality as the embodiment shown in Figure 2. The fixation sections 5B are however represented by tabs (cf. Figure 5) formed at the second interface component 2, each of which comprises a hooked protrusion adapted to snap into correspondingly formed recesses 5A at the first interface component 1. While the first interface component 1 of Figures 1, 2 and 3 may be formed from a metallic material in a milling process, the second interface component 2 of Figure 3 is a injection-molded-product made from a plastic material. Therefore, the tabs 5B can deform elastically and may therefore deflect while the second interface component 2 is pressed onto the first interface component 1 until the hook-shaped protrusions of tabs 5B snap into the recesses 5A, thereby holding the interface components 1 and 2 firmly together.

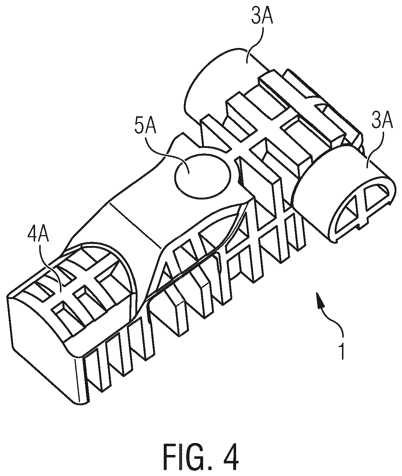

The first interface component 1 shown in Figure 4 has the same functionality as the first interface components 1 shown in Figures 2 and 3. It is however formed as a injection-molded-product made from a plastic material.

Figure 5 shows a 4-side-view of the injection-molded second interface component 2 of Figure 3.

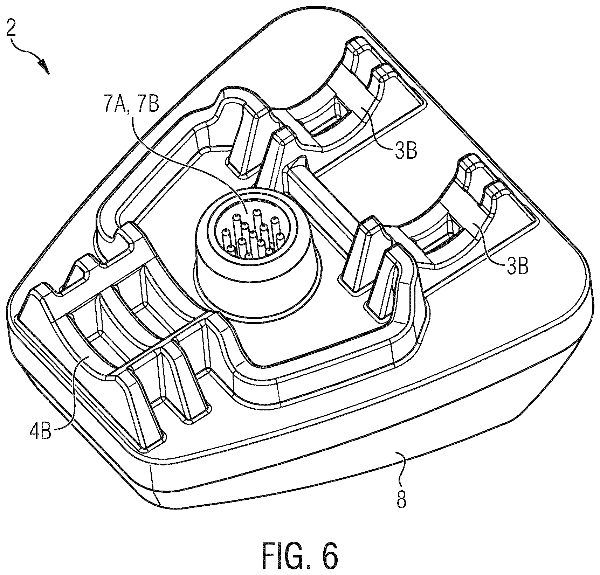

Figure 6 shows another embodiment of a second interface component 2 which additionally comprises a signal- and/or power-section 7A, 7B that provides a plurality of electrical contacts for transmitting electric power and/or signals to and/or from an electromagnetic (EM)-sensor 8 to be used in conjunction with an EM-tracking-system. In the embodiment shown in Figure 6, no fixation sections 5A and 5B are shown for the sake of clarity of presentation.

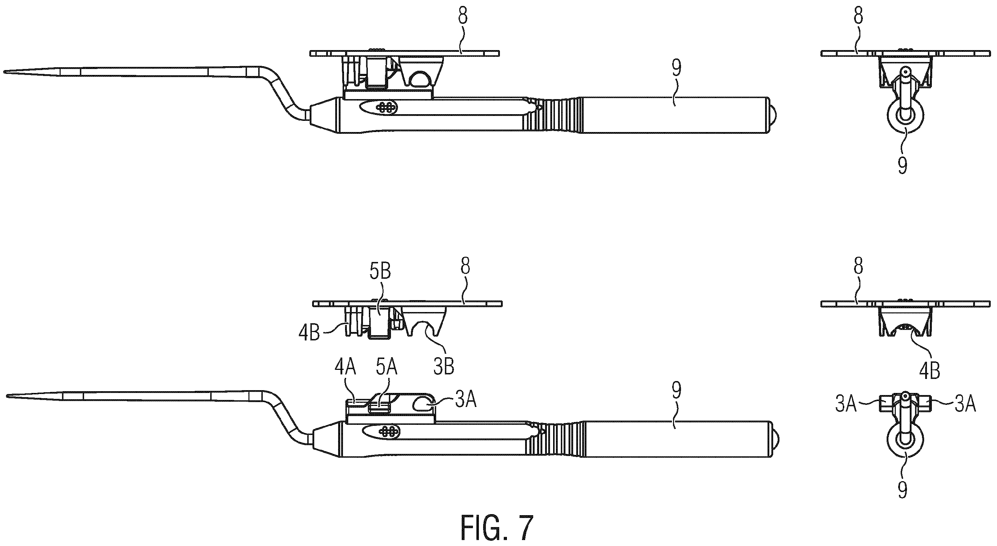

Figure 7 shows the pointer instrument of Figure 1 along with a planar (2D-)tracking marker 8, both of which interconnect via tracking marker interface components.

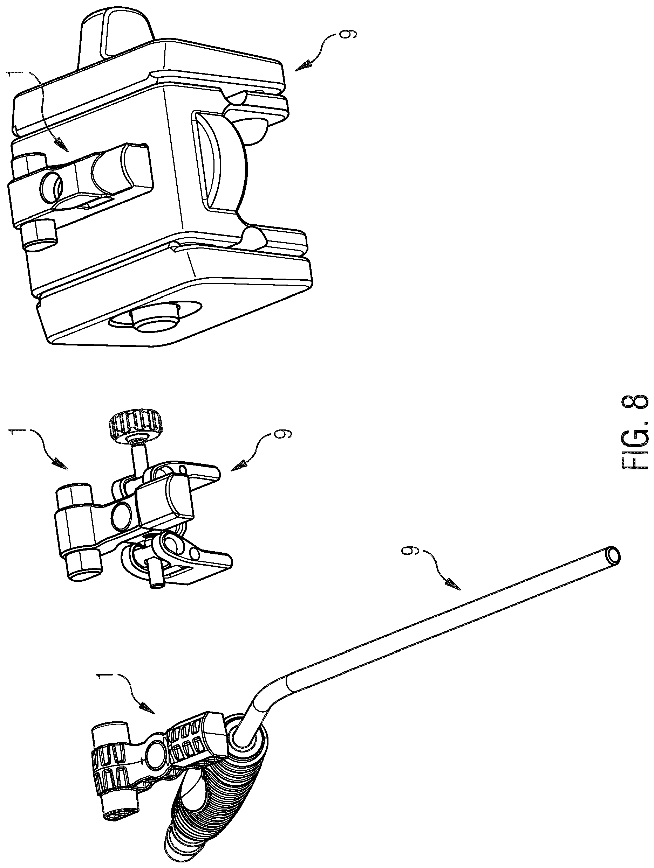

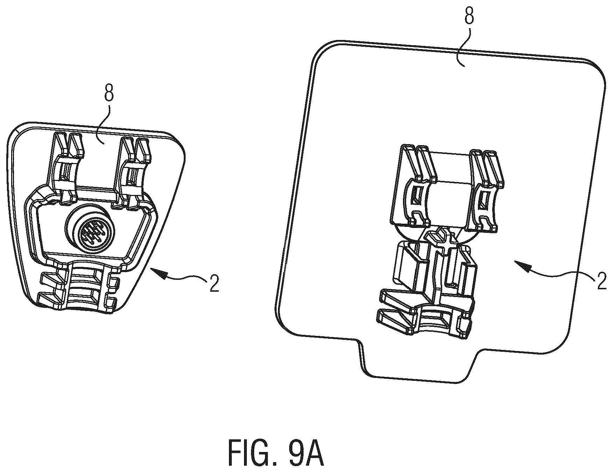

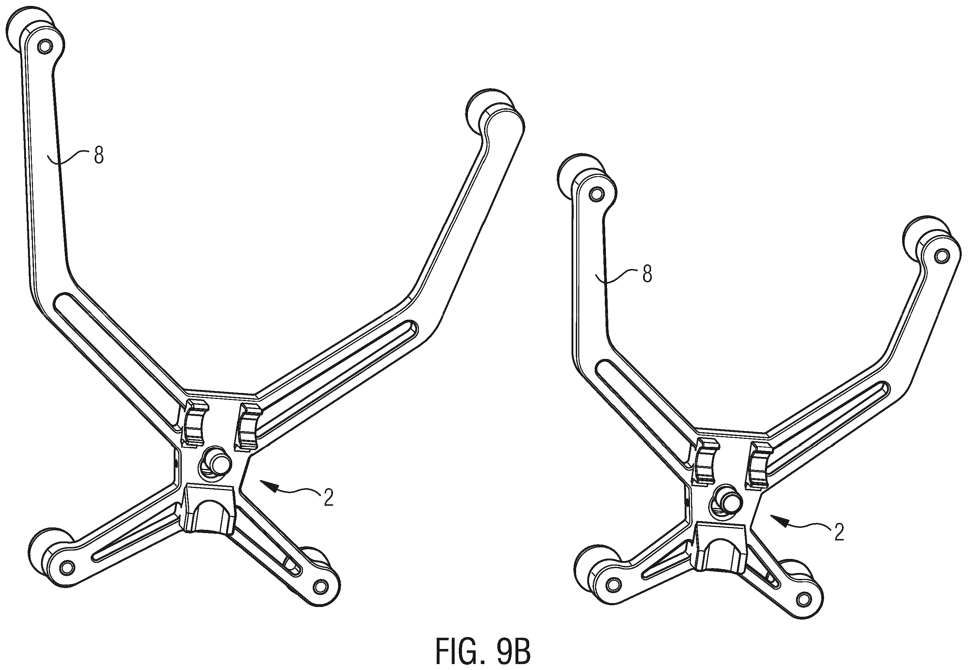

The inventive tracking marker interface can be used for any conceivable instrument or device the spatial position of which needs to be determined during a medical or surgical procedure, a selection of which is shown in Figure 8. It needs to be noted that each one of the instruments and devices 9 shown in Figure 8 features a first interface component 1 that provides positioning sections 3A and 4A with the same geometry and relative position. Additionally, each one of the interface components also comprises one or more fixation section 5A and 5B as described herein. This allows for connecting a specific type of tracking marker 8 to any instrument or device featuring the first interface component 1. On the other hand, each of the various tracking markers 8 shown for example in Figure 1 and Figures 9A and 9B features a second interface component 2 providing the same first and second positioning sections 3B and 4B and at least one of the fixation sections 5B described herein. Thus, any of the tracking markers shown in Figures 1 or Figure 9A and 9B can be releasably attached to any of the instruments shown in Figure 1 and Figure 8.

## Claims

1. Tracking marker interface component (2) comprising at least one first positioning section (3B), at least one second positioning section (4B) and at least one fixation section (5B), wherein
- the fixation section (5B) is adapted to retain the interface component (2) and to establish a positive fit and/or a friction fit along a first spatial axis (Z);
- the first positioning section (3B) features a profile extending along a third spatial axis (Y) perpendicular to the first spatial axis (Z); **characterized in that**
- the second positioning section (4B) features a profile extending along a second spatial axis (X) perpendicular to the first spatial axis (Z) and to the third spatial axis (Y).

2. Tracking marker interface component according to claim 1, wherein the first positioning section (3B) and/or the second positioning section (4B) is/are shaped as a protrusion protruding from the interface component (1, 2), particularly along the first spatial axis (Z).

3. Tracking marker interface component according to claim 1, wherein the first positioning section (3B) and/or the second positioning section (4B) is/are shaped as a recess indenting the interface component (2), particularly along the first spatial axis (Z).

4. Tracking marker interface component according to one of claims 2 and 3, wherein the protrusion comprises at least one convex, particularly cylindrically shaped surface section and/or wherein the recess comprises at least one concave or prismatically shaped surface section.

5. Tracking marker interface component according to one of claims 1 to 4, wherein the fixation section (5B) includes
- an external or an internal thread; and/or
- a connector portion having an undercut extending perpendicularly to the first spatial axis (Z); and/or
- a permanent magnet or an electromagnet.

6. Tracking marker interface component according to any one of claims 1 to 5, wherein the interface component (2) connects to or is integrally formed with a tracking marker (8) adapted to be recognized and spatially tracked by a medical tracking system.

7. Tracking marker interface component according to any one of claims 1 to 6, wherein at least one of the first positioning section (3B) and the second positioning section (4B) comprises an at least sectionwise constant profile along the third spatial axis (Y) and the second spatial axis (X), respectively.

8. Tracking marker interface component according to any one of claims 1 to 7, wherein at least one of the first positioning section (3B) and the second positioning section (4B) comprises at least two sections having an identical profile along the third spatial axis (Y) and the second spatial axis (X), respectively.

9. Tracking marker interface component according to any one of claims 1 to 8, wherein the fixation section (5B) is disposed between the first positioning section (3B) and the second positioning section (4B).

10. Tracking marker interface component according to any one of claims 1 to 9, wherein the interface component (2) is configured as disposable article and comprises, particularly at the fixation section (5B), a predetermined breaking section that disables functionality of the interface component (2), particularly of the fixation section (5B), when being fractionized.

11. Tracking marker interface component according to claim 10, wherein the predetermined breaking section is adapted to be fractionized by
- a force and/or a moment exceeding a predefined amount and acting on the fixation section (5B) along the first spatial axis (Z);
- a force and/or a moment exceeding a predefined amount and acting on the fixation section (5B) in a direction opposite to an engagement direction in which the fixation section (5B) is configured to move for establishing the positive fit and/or the friction fit.

12. Tracking marker interface component according to any one of claims 10 and 11, wherein the breaking section connects between the interface component (2), particularly the fixation section (5B), and an element configured to be detached from the interface component (2), particularly the fixation section (5B), wherein the detachable element is in particular adapted to be torn off or twisted off the interface component (2), particularly the fixation section (5B).

13. Tracking marker interface component according to claim 12, wherein the detachable element is configured to prevent the fixation section (5B) to move in a direction opposite to an engagement direction in which the fixation section (5B) is configured to move for establishing the positive fit and/or the friction fit.

14. Tracking marker interface component according to any one of claims 1 to 13, which is injection molded and particularly configured
- as undercut-free structure;
- to be molded in a slider-free injection-mold;
- to be molded in an injection-mold having one single unmolding direction; and/or
- to be demolded without elastic deformation.

15. Use of the tracking marker interface component according to any one of claims 1 to 14 for connecting, particularly in a statically determined manner, a medical tracking marker to a medical instrument or medical device, particularly by bringing the tracking marker interface component (2) into contact with a correspondingly shaped counterpart interface component (1).

## Patentansprüche

1. Trackingmarker-Schnittstellenkomponente (2), die mindestens einen ersten Positionierungsabschnitt (3B), mindestens einen zweiten Positionierungsabschnitt (4B) und mindestens einen Befestigungsabschnitt (5B) umfasst, wobei
- der Fixierungsabschnitt (5B) dazu ausgelegt ist, die Schnittstellenkomponente (2) zu halten und eine formschlüssige und/oder reibschlüssige Verbindung entlang einer ersten Raumachse (Z) herzustellen;
- der erste Positionierungsabschnitt (3B) ein Profil aufweist, das sich entlang einer dritten Raumachse (Y) senkrecht zur ersten Raumachse (Z) erstreckt; **dadurch gekennzeichnet, dass**
- der zweite Positionierungsabschnitt (4B) ein Profil aufweist, das sich entlang einer zweiten Raumachse (X) senkrecht zur ersten Raumachse (Z) und zur dritten Raumachse (Y) erstreckt.

2. Schnittstellenkomponente für Trackingmarker gemäß Anspruch 1, wobei der erste Positionierungsabschnitt (3B) und/oder der zweite Positionierungsabschnitt (4B) als Vorsprung ausgebildet ist/sind, der von der Schnittstellenkomponente (1, 2) vorsteht, insbesondere entlang der ersten Raumachse (Z).

3. Trackingmarker-Schnittstellenkomponente nach Anspruch 1, wobei der erste Positionierungsabschnitt (3B) und/oder der zweite Positionierungsabschnitt (4B) als eine in die Schnittstellenkomponente (2) insbesondere entlang der ersten Raumachse (Z) hineinragende Vertiefung ausgebildet ist/sind.

4. Trackingmarker-Schnittstellenkomponente nach einem der Ansprüche 2 und 3, wobei der Vorsprung mindestens einen konvexen, insbesondere zylindrisch geformten Oberflächenabschnitt umfasst und/oder wobei die Vertiefung mindestens einen konkaven oder prismatisch geformten Oberflächenabschnitt umfasst.

5. Trackingmarker-Schnittstellenkomponente nach einem der Ansprüche 1 bis 4, wobei der Befestigungsabschnitt (5B) umfasst
- ein Außen- oder Innengewinde; und/oder
- einen Verbindungsabschnitt mit einer senkrecht zur ersten Raumachse (Z) verlaufenden Hinterschneidung; und/oder
- einen Permanentmagneten oder einen Elektromagneten.

6. Schnittstellenkomponente für einen Trackingmarker gemäß einem der Ansprüche 1 bis 5, wobei die Schnittstellenkomponente (2) mit einem Trackingmarker (8) verbunden oder integral mit diesem ausgebildet ist, der so ausgelegt ist, dass er von einem medizinischen Trackingsystem erkannt und räumlich verfolgt werden kann.

7. Trackingmarker-Schnittstellenkomponente nach einem der Ansprüche 1 bis 6, wobei mindestens einer der ersten Positionierungsabschnitte (3B) und der zweiten Positionierungsabschnitte (4B) ein mindestens abschnittsweise konstantes Profil entlang der dritten Raumachse (Y) bzw. der zweiten Raumachse (X) aufweist.

8. Trackingmarker-Schnittstellenkomponente nach einem der Ansprüche 1 bis 7, wobei mindestens einer der ersten Positionierungsabschnitte (3B) und der zweiten Positionierungsabschnitte (4B) mindestens zwei Abschnitte mit einem identischen Profil entlang der dritten Raumachse (Y) bzw. der zweiten Raumachse (X) umfasst.

9. Trackingmarker-Schnittstellenkomponente nach einem der Ansprüche 1 bis 8, wobei der Fixierungsabschnitt (5B) zwischen dem ersten Positionierungsabschnitt (3B) und dem zweiten Positionierungsabschnitt (4B) angeordnet ist.

10. Trackingmarker-Schnittstellenkomponente nach einem der Ansprüche 1 bis 9, wobei die Schnittstellenkomponente (2) als Einwegartikel ausgebildet ist und insbesondere am Fixierungsabschnitt (5B) eine Sollbruchstelle aufweist, der die Funktionalität der Schnittstellenkomponente (2), insbesondere des Fixierungsabschnitts (5B), bei Bruch außer Kraft setzt.

11. Trackingmarker-Schnittstellenkomponente nach Anspruch 10, wobei der vorbestimmte Sollbruchstelle so ausgelegt ist, dass er durch
- eine Kraft und/oder ein Moment, die einen vorbestimmten Wert überschreiten und entlang der ersten Raumachse (Z) auf den Befestigungsabschnitt (5B) einwirken;
- eine Kraft und/oder ein Moment, die einen vordefinierten Wert überschreiten und in einer Richtung auf den Befestigungsabschnitt (5B) einwirken, die der Eingriffsrichtung entgegengesetzt ist, in der sich der Befestigungsabschnitt (5B) bewegen soll, um den Formschluss und/oder Reibschluss herzustellen.

12. Trackingmarker-Schnittstellenkomponente nach einem der Ansprüche 10 und 11, wobei die Sollbruchstelle zwischen der Schnittstellenkomponente (2), insbesondere dem Befestigungsabschnitt (5B), und einem Element angeordnet und mit diesen verbunden ist, das so konfiguriert ist, dass es von der Schnittstellenkomponente (2), insbesondere dem Befestigungsabschnitt (5B), gelöst werden kann, wobei das lösbare Element insbesondere so ausgelegt ist, dass es von der Schnittstellenkomponente (2), insbesondere dem Befestigungsabschnitt (5B) abgerissen oder abgedreht werden kann.

13. Trackingmarker-Schnittstellenkomponente gemäß Anspruch 12, wobei das abnehmbare Element so konfiguriert ist, dass es verhindert, dass sich der Befestigungsabschnitt (5B) in einer Richtung entgegengesetzt zu einer Eingriffsrichtung bewegt, in der sich der Befestigungsabschnitt (5B) bewegt, um den Formschluss und/oder den Reibschluss herzustellen.

14. Trackingmarker-Schnittstellenkomponente gemäß einem der Ansprüche 1 bis 13, die spritzgegossen und insbesondere als folgendes ausgestaltet ist:
- als hinterschneidungsfreie Struktur;
- zur Herstellung in einer schlittenfreien Spritzgussform;
- zur Herstellung in einer Spritzgussform mit einer einzigen Entformungsrichtung; und/oder
- zum Entformen ohne elastische Verformung.

15. Verwendung der Trackingmarker-Schnittstellenkomponente gemäß einem der Ansprüche 1 bis 14 zum Verbinden, insbesondere in statisch festgelegter Weise, eines medizinischen Trackingmarkers mit einem medizinischen Instrument oder einer medizinischen Vorrichtung, insbesondere durch in Kontakt bringen der Trackingmarker-Schnittstellenkomponente (2) mit einer entsprechend geformten Gegenstück-Schnittstellenkomponente (1).

## Revendications

1. Composant d'interface de marqueur de suivi (2) comprenant au moins une première section de positionnement (3B), au moins une deuxième section de positionnement (4B) et au moins une section de fixation (5B), étant entendu que
- la section de fixation (5B) est adaptée à retenir le composant d'interface (2) et à établir un ajustement par complémentarité de forme et/ou un ajustement par frottement le long d'un premier axe spatial (Z),
- la première section de positionnement (3B) présente un profil s'étendant le long d'un troisième axe spatial (Y) perpendiculaire au premier axe spatial (Z) ; **caractérisé en ce que**
- la deuxième section de positionnement (4B) présente un profil s'étendant le long d'un deuxième axe spatial (X) perpendiculaire au premier axe spatial (Z) et au troisième axe spatial (Y).

2. Composant d'interface de marqueur de suivi selon la revendication 1, dans lequel la première section de positionnement (3B) et/ou la deuxième section de positionnement (4B) ont la forme d'une saillie ressortant du composant d'interface (1, 2), notamment le long du premier axe spatial (Z).

3. Composant d'interface de marqueur de suivi selon la revendication 1, dans lequel la première section de positionnement (3B) et/ou la deuxième section de positionnement (4B) ont la forme d'un évidement en renfoncement dans le composant d'interface (2), notamment le long du premier axe spatial (Z).

4. Composant d'interface de marqueur de suivi selon l'une quelconque des revendications 2 et 3, dans lequel la saillie comprend au moins une section de surface convexe, notamment de forme cylindrique, et/ou l'évidement comprend au moins une section de surface concave ou de forme prismatique.

5. Composant d'interface de marqueur de suivi selon l'une quelconque des revendications 1 à 4, dans lequel la section de fixation (5B) comprend
- un filetage externe ou interne, et/ou
- une portion de raccordement présentant une contre-dépouille s'étendant perpendiculairement au premier axe spatial (Z), et/ou
- un aimant permanent ou un électroaimant.

6. Composant d'interface de marqueur de suivi selon l'une quelconque des revendications 1 à 5, dans lequel le composant d'interface (2) se raccorde à un marqueur de suivi (8) adapté à être reconnu et suivi spatialement par un système de suivi médical, ou est formé d'un seul tenant avec celui-ci.

7. Composant d'interface de marqueur de suivi selon l'une quelconque des revendications 1 à 6, dans lequel au moins une des première section de positionnement (3B) et deuxième section de positionnement (4B) comprend un profil constant au moins par sections respectivement le long du troisième axe spatial (Y) et du deuxième axe spatial (X).

8. Composant d'interface de marqueur de suivi selon l'une quelconque des revendications 1 à 7, dans lequel au moins une des première section de positionnement (3B) et deuxième section de positionnement (4B) comprend au moins deux sections présentant un profil identique respectivement le long du troisième axe spatial (Y) et du deuxième axe spatial (X).

9. Composant d'interface de marqueur de suivi selon l'une quelconque des revendications 1 à 8, dans lequel la section de fixation (5B) est disposée entre la première section de positionnement (3B) et la deuxième section de positionnement (4B).

10. Composant d'interface de marqueur de suivi selon l'une quelconque des revendications 1 à 9, dans lequel le composant d'interface (2) est conçu comme un article jetable et comprend, notamment au niveau de la section de fixation (5B), une section de rupture prédéterminée qui désactive la fonctionnalité du composant d'interface (2), notamment de la section de fixation (5B), lorsqu'elle est fractionnée.

11. Composant d'interface de marqueur de suivi selon la revendication 10, dans lequel la section de rupture prédéterminée est adaptée à être fractionnée sous l'effet de
- une force et/ou un moment dépassant une valeur prédéfinie et agissant sur la section de fixation (5B) le long du premier axe spatial (Z),
- une force et/ou un moment dépassant une valeur prédéfinie et agissant sur la section de fixation (5B) dans une direction opposée à une direction d'accouplement dans laquelle la section de fixation (5B) est conçue pour se déplacer afin d'établir l'ajustement par complémentarité de forme et/ou l'ajustement par frottement.

12. Composant d'interface de marqueur de suivi selon l'une quelconque des revendications 10 et 11, dans lequel la section de rupture raccorde le composant d'interface (2), notamment la section de fixation (5B), et un élément conçu pour être détaché du composant d'interface (2), notamment de la section de fixation (5B), l'élément détachable étant notamment adapté à être arraché du composant d'interface (2) ou retiré de celui-ci par torsion, notamment de la section de fixation (5B).

13. Composant d'interface de marqueur de suivi selon la revendication 12, dans lequel l'élément détachable est conçu pour empêcher la section de fixation (5B) de se déplacer dans une direction opposée à une direction d'accouplement dans laquelle la section de fixation (5B) est conçue pour se déplacer afin d'établir l'ajustement par complémentarité de forme et/ou l'ajustement par frottement.

14. Composant d'interface de marqueur de suivi selon l'une quelconque des revendications 1 à 13, qui est moulé par injection et conçu notamment
- sous la forme d'une structure sans contre-dépouille,
- pour être moulé dans un moule à injection sans coulisseau,
- pour être moulé dans un moule à injection présentant une seule direction de démoulage, et/ou
- pour être démoulé sans déformation élastique.

15. Utilisation du composant d'interface de marqueur de suivi selon l'une quelconque des revendications 1 à 14 pour raccorder, notamment d'une manière déterminée statiquement, un marqueur de suivi médical à un instrument médical ou à un dispositif médical, notamment par mise en contact du composant d'interface de marqueur de suivi (2) avec un composant d'interface (1) homologue de forme correspondante.
